# FASCICULE DE BREVET EUROPEEN

(11) **EP 3 642 323 B1**
(45) Date de publication et mention de la délivrance du brevet: **18.10.2023**
(21) Numéro de dépôt: 18725288.7
(22) Date de dépôt: 25.04.2018
(51) Int. Cl.: C12N 1/04

(54) **PROCEDE DE CONSERVATION D'UN ECHANTILLON DE BACTERIES**
VERFAHREN ZUR KONSERVIERUNG EINER BAKTERIENPROBE
METHOD FOR PRESERVING A SAMPLE OF BACTERIA

(30) Priorité: 23.06.2017 FR 1755796
(43) Date de publication de la demande: 29.04.2020
(73) Titulaire: Fondation Méditerranée Infection, 13005 Marseille (FR); Université d'Aix-Marseille, 13284 Marseille Cedex 07 (FR); Assistance Publique - Hôpitaux de Marseille, 13005 Marseille (FR); Centre National de la Recherche Scientifique (CNRS), 75016 Paris (FR); INSERM (Institut National de la Santé et de la Recherche Médicale), 75013 Paris (FR)
(72) Inventeur: LAGIER, Jean-Christophe, 13008 Marseille (FR); RAOULT, Didier, 13008 Marseille (FR); BELLALI, Sara, 13005 Marseille (FR); KHELAIFIA, Saber, 13010 Marseille (FR); GOUIRAN, Camille, 13500 Martigues (FR); CHABRIERE, Eric, F-13009 Marseilles (FR)
(74) Mandataire: Cabinet Beau de Loménie
(86) Numéro de dépôt international: PCT/FR2018/051036
(87) Numéro de publication internationale: WO 2018/234645

(56) Documents cités:
- WO-A1-2015/159004
- WO-A1-2017/103225
- FREDERIEK-MAARTEN KERCKHOF ET AL: "Optimized Cryopreservation of Mixed Microbial Communities for Conserved Functionality and Diversity", PLOS ONE, vol. 9, no. 6, 17 juin 2014 (2014-06-17), page e99517, XP055265294, DOI: 10.1371/journal.pone.0099517
- LIN ENMOORE ET AL: "Twelve Week Storage Trial of Microbial Viability in Lyophilized and Frozen Fecal Microbiota Preparations", GASTROENTEROLOGY, W.B. SAUNDERS CO, US, vol. 148, no. 4, Suppl.1, 1 mai 2014 (2014-05-01), page S962, XP009189480, ISSN: 0016-5085
- MARISOL AGUIRRE ET AL: "Evaluation of an optimal preparation of human standardized fecal inocula for in vitro fermentation studies", JOURNAL OF MICROBIOLOGICAL METHODS, vol. 117, 1 octobre 2015 (2015-10-01), pages 78-84, XP055272416, NL ISSN: 0167-7012, DOI: 10.1016/j.mimet.2015.07.019
- HUBÁLEK Z: "Protectants used in the cryopreservation of microorganisms", CRYOBIOLOGY, ACADEMIC PRESS INC, US, vol. 46, no. 3, 1 juin 2003 (2003-06-01), pages 205-229, XP002373087, ISSN: 0011-2240, DOI: 10.1016/S0011-2240(03)00046-4

## Description

La présente invention concerne un procédé de conservation d'un échantillon d'un ensemble de bactéries aérobies et anaérobies autres que des bactéries lactiques, notamment des bactéries du microbiote intestinal, plus particulièrement encore un échantillon de selles humaines, et un lyophilisat obtenu.

Les selles humaines se caractérisent par la présence d'un microbiote intestinal comprenant l'ensemble des microorganismes du tube digestif humain notamment des bactéries aérobies et bactéries anaérobies strictes et des archaea methanogènes telles que *Methanobrevibacter smithii* ces dernières étant les plus difficiles à conserver.

La méthode la plus efficace à ce jour de conservation d'échantillons de selles préservant la viabilité des bactéries est la congélation, mais ce mode de conservation est peu pratique et couteux de par la nécessité de maintenir la chaîne du froid pendant le transport et stockage.

La lyophilisation vise à retirer l'eau contenue dans un produit par congélation suivi d'une sublimation pour en faciliter la conservation. Les produits congelés sont ainsi séchés sous vide, sans dégel.

La lyophilisation de selles humaines permet une conservation dans des conditions évitant la chaîne du froid et permettant leur mise en gélule pour être administrée à usage thérapeutique notamment dans le cadre de traitements probiotiques ou de greffes de microbiote intestinal par lesquelles on fait absorber les microbes d'une selle de sujet sain par un patient porteur d'une colite ou d'une infection par une bactérie multi résistante telle que Clostridium difficile. Idéalement, un échantillon fécal autologue est prélevé chez le patient avant le traitement médical, par exemple avant une opération ou une situation à risque. L'échantillon est conservé. Si le patient vient à développer une infection à C. difficile, alors l'échantillon est extrait dans une solution saline et filtré. Le filtrat est lyophilisé. Le lyophilisat (poudre) résultant est mis sous forme de gélules entérosolubles (résistantes à l'acide gastrique stomacal) pour restaurer la flore colique antérieure du patient en faisant concurrence à C. difficile. Cette procédure permet d'éviter les risques d'introduire un autre pathogène provenant d'un donneur qui n'est pas le patient. Elle permet aussi d'éviter la problématique de l'administration par sonde nasale pour atteindre le duodénum du patient.

La lyophilisation entraîne toutefois la destruction ou diminution de la viabilité d'un nombre considérable de bactéries avec une mortalité bactérienne qui va de 90% à 99.99% en fonction de leur caractère aérotolérant ou aérointolérant.

On connaît des procédés de lyophilisation de bactéries visant à en améliorer la viabilité avec des agents protecteurs mais ceux-ci varient selon le type de bactéries et ne concernent pas la lyophilisation d'échantillons cliniques de selles.

La lyophilisation est un procédé de séchage à froid en général utilisé uniquement pour les échantillons bactérien de bactéries très sensibles à la chaleur comme les bactéries lactiques. Dans WO 2003/018778 on améliore la viabilité de bactéries lactiques lyophilisée en ajoutant dans l'échantillon avant lyophilisation des agents protecteurs comprenant :
- un composé antioxydant, et
- un composé augmentant la température de transition vitreuse du lyophilisat à savoir du glycérol ou un hydrate de carbone tel que lactose, galactose, maltose, saccharose et glucose.

Selon la présente invention, on a démontré que ces conditions ne sont pas suffisantes pour améliorer suffisamment la viabilité des bactéries de l'ensemble du microbiote intestinal, notamment dans des échantillons cliniques de selles humaines.

Le but de la présente invention est de fournir un procédé pour conserver la viabilité maximale des bactéries du microbiote intestinale dans des selles humaines.

Selon la présente invention on a découvert des conditions uniques communes de lyophilisation pour multiplier par 100 au moins le nombre des bactéries aérobies aussi bien que bactéries anaérobies viables présentes dans un échantillon d'un ensemble de dites bactéries aérobies et anaérobies autres que des bactéries lactiques, notamment un échantillon de selles humaines après lyophilisation en comparaison au procédé standard de lyophilisation.

Plus précisément, selon la présente invention ces conditions de lyophilisation consistent à :
- d'une part, diluer les échantillons bactériens, notamment échantillon de selles dans un milieu aqueux de conservation comprenant une combinaison spécifique d'agent(s) protecteur(s), et
- d'autre part, mettre en oeuvre une étape initiale de congélation très rapide dans l'azote liquide à -196°C environ avant de réaliser le procédé de lyophilisation. Le procédé de lyophilisation impliquant une congélation suivie d'une étape de déshydratation par sublimation. Ces conditions ont permis d'obtenir les meilleurs résultats de viabilité comparés aux méthodes connues de conservation par congélation à - 80°C ou de lyophilisation par simple ajout d'agent lyoprotecteurs conventionnels.

Alors que les cellules congelées rapidement ont généralement un taux de survie plus faible que les cellules congelées lentement, selon la présente invention, on a découvert de façon surprenante que -sous réserve de respecter un milieu de conservation spécifique visé ci-dessus, la congélation rapide dans l'azote (-196°C) permet d'augmenter le taux de survie des dites bactéries, notamment du microbiote de selles humaines d'un facteur 100 au moins par rapport à une congélation lente à -80°C avant lyophilisation.

Pour ce faire, la présente invention fournit un procédé de conservation d'un échantillon d'un ensemble de bactéries aérobies et anaérobies autres que des bactéries lactiques, notamment des bactéries du microbiote intestinal, comprenant les étapes successives suivantes dans lesquelles :
a) on prépare un dit échantillon d'un ensemble de dites bactéries par dilution dans un milieu aqueux de conservation comprenant une combinaison d'agents protecteurs consistant en (a1) une combinaison de composés antioxydants choisis parmi de l'acide ascorbique, de l'acide urique et du glutathion, (a2) au moins 2 sucres différents consistant en du saccharose et du tréhalose, et (a3) des protéines de lait, et
b) on réalise une congélation dudit échantillon récupéré à l'étape a) dans de l'azote liquide à environ -196°C pendant pas plus de 2 minutes, de préférence pas plus de 1 minute, notamment pendant 30 à 50 secondes, et
c) on réalise une lyophilisation du dit échantillon ainsi congelé pour obtenir un lyophilisat.

A l'étape b), on réalise une congélation dudit échantillon récupéré à l'étape a) dans un récipient, par immersion du récipient contenant ledit échantillon dans de l'azote liquide.

On entend ici par « protéines de lait », les protéines choisies parmi principalement des caséines et les protéines lactosériques comme la lactalbumine et les lactoglobulines.

A l'étape c), la lyophilisation implique une sublimation consistant en une déshydratation du produit congelé par dessiccation sous vide à froid de la glace sans le faire fondre, l'eau se sublime c'est-à-dire qu'elle passe directement de l'état solide à gazeux. Cette étape consiste donc essentiellement ici en l'établissement d'un vide poussé.

Plus particulièrement, à l'étape c) l'échantillon congelé est déposé dans un lyophilisateur pendant au moins 12h à une température d'au moins 0°C et à une pression de vide poussé de pas plus de 1mbar.

Plus particulièrement, à l'étape a), on prépare un dit échantillon qui est un échantillon clinique de selles par dilution desdites selles dans undit milieu aqueux de conservation, et on filtre la dispersion obtenue à pour en retirer au moins les débris de tailles supérieures à 2mm.

De préférence, à l'étape a), on dilue ledit échantillon dans un milieu aqueux de conservation comprenant au moins la triple combinaison (a1) d'au moins un composé antioxydant de préférence au moins deux composés antioxydants et (a2) au moins deux sucres différents, et (a3) des protéines de lait.

Plus particulièrement, les protéines de lait sont sous forme de lait entier, de préférence à une concentration pondérale dans le dit milieu de conservation d'au moins 5%, de préférence 10%.

Plus particulièrement encore, les dits agents protecteurs comprennent une combinaison de composés antioxydants choisi parmi l'acide ascorbique, l'acide urique et le glutathion.

Plus particulièrement encore, les dits agents protecteurs comprennent une combinaison de composés antioxydants consistant dans de l'acide ascorbique à une concentration de 1g/L, de l'acide urique à une concentration de 0,4g/L et du glutathion à une concentration de 0,1g/L.

Plus particulièrement encore, les dits agents protecteurs comprennent au moins un sucre choisi parmi le saccharose (aussi dénommé ci-après « sucrose ») et le tréhalose.

Plus particulièrement encore, dits agents protecteurs comprennent du saccharose à une concentration pondérale dans le dit milieu de conservation d'au moins 5%, de préférence 10%.

Plus particulièrement encore, le dit milieu de conservation est un milieu tampon à PH de 7 à 7.5 comprenant dans de l'eau les sels KCL, CaCl₂, MgCl₂, KH₂PO₄, Na₂HPO₄, NaCL et KOH ainsi qu'au moins un dit agent protecteur.

Plus particulièrement encore, le dit milieu de conservation comprend :
(a1) une combinaison de composés antioxydants consistant dans de l'acide ascorbique à une concentration de 1g/L, de l'acide urique à une concentration de 0,4g/L et du glutathion à une concentration de 0,1g/L, et
(a2) du saccharose à une concentration pondérale dans le dit milieu de conservation de 5%, et du tréhalose à une concentration pondérale dans le dit milieu de conservation de 10%, et
(a3) des protéines de lait à une concentration pondérale dans le dit milieu de conservation de 10%.

Plus particulièrement encore, le dit échantillon comprend au moins les bactéries aérobies et bactéries anaérobies suivantes : *Staphylococcus aureus, Enterobacter aerogenes, Escherichia coli, Klebsiella oxytoca, Streptococcus agalactiae, Serratia marcescens, Proteus mirabilis, Staphylococcus epidermidis, Bacillus circulans, Streptococcus pneumoniae, Staphylococcus hominis et Acinetobacter baumanii.*

Plus particulièrement encore, le dit échantillon comprend au moins les bactéries anaérobies strictes suivantes : *Bacteroides fragilis, Bacteroides nordii, Bacteroides thetaiotaomicron, Clostidium butyricum, Clostridium massilioamazoniensis, Clostridium beijerinckii, Clostridium irregular Finegoldia magna, Propionibacterium acnes, Propionibacterium avidum, Haemophilus influenzae.*

La présente invention a donc également pour objet un lyophilisat d'échantillon d'un ensemble de bactéries aérobies et anaérobies autres que des bactéries lactiques, notamment des bactéries du microbiote intestinal, plus particulièrement encore un échantillon de selles humaines, obtenu par un procédé selon l'invention tel que défini ci-dessus, caractérisé en ce qu'il comprend :
- au moins un agent protecteur consistant dans au moins la combinaison d'agents protecteurs consistant dans (a1) une combinaison de composés antioxydants choisis parmi de l'acide ascorbique, de l'acide urique et du glutathion, (a2) au moins 2 sucres différents consistant en du saccharose et du tréhalose, et (a3) des protéines de lait, et
- une quantité de bactéries du microbiote intestinal vivantes de préférence au nombre de :
   - au moins 10⁸ CFU/mL bactéries de chacune des catégories des bactéries aérobies et respectivement bactéries anaérobies mises en culture après 48h de conservation à 4°C, et
   - au moins 10⁸ CFU/mL bactéries de chacune des catégories des bactéries aérobies et respectivement bactéries anaérobies mise en culture après 6 semaines de conservation à 4°C.

Plus particulièrement, un lyophilisat selon l'invention comprend des dits agents protecteurs consistant dans a1) une combinaison de 3 composés antioxydants consistant dans de l'acide ascorbique, de l'acide urique et du glutathion, et a2) au moins 2 sucres différents consistant dans du saccharose et du tréhalose, et a3) des protéines de lait.

D'autres caractéristiques et avantages de la présente invention ressortiront mieux à la lecture de la description détaillée qui va suivre, faite de manière illustrative et non limitative, en référence aux dessins annexés sur lesquels :

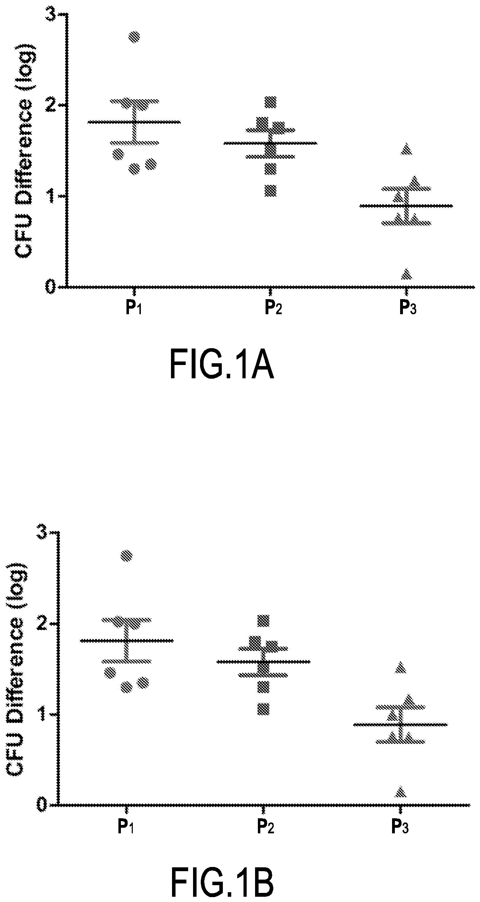

- les figures 1A et 1B illustrent les résultats de différences de quantité de bactéries vivantes détectées par les différences de nombres de log de CFU de bactéries aérobies (figure 1A) et bactéries anaérobies (figure 1B) après différents protocoles de congélation utilisés P1,P2 etP3 sur des échantillons de selles sans agent protecteur dans le milieu de conservation ;

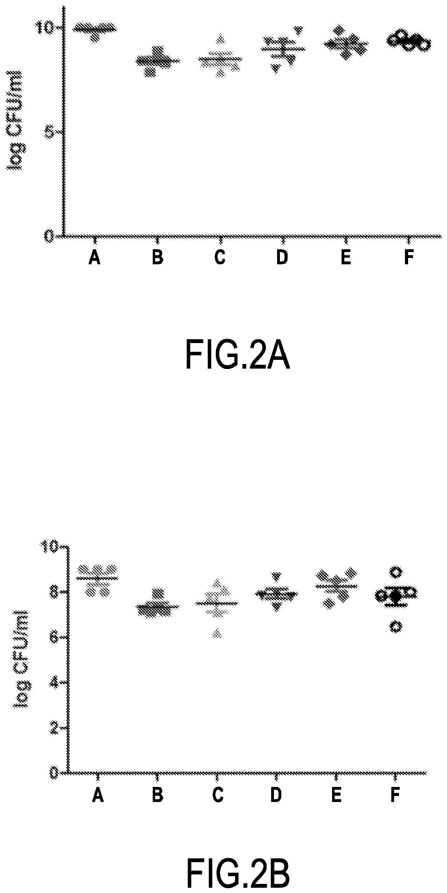

- les figures 2A et 2B illustrent les résultats de quantité de bactéries vivantes détectées en anaérobiose à 48 heures (figure 2A) et en anaérobiose après 6 semaines de conservation du lyophilisat à 4°C (figure 2B), par le log CFU/mL, sur des échantillons de selles avec différents conditions de milieux de conservations : A= selles fraîches dans eau saline, B= lyophilisat dans milieu PBS, C= Lyophilisat avec milieu PBS contenant des antioxydants seuls, D= Lyophilisat avec milieu PBS contenant du lait en poudre seul, E= Lyophilisat avec milieu PBS contenant antioxydants + saccharose + lait en poudre , F= congélation de selle fraîches dans milieu PBS sans agent protecteur;

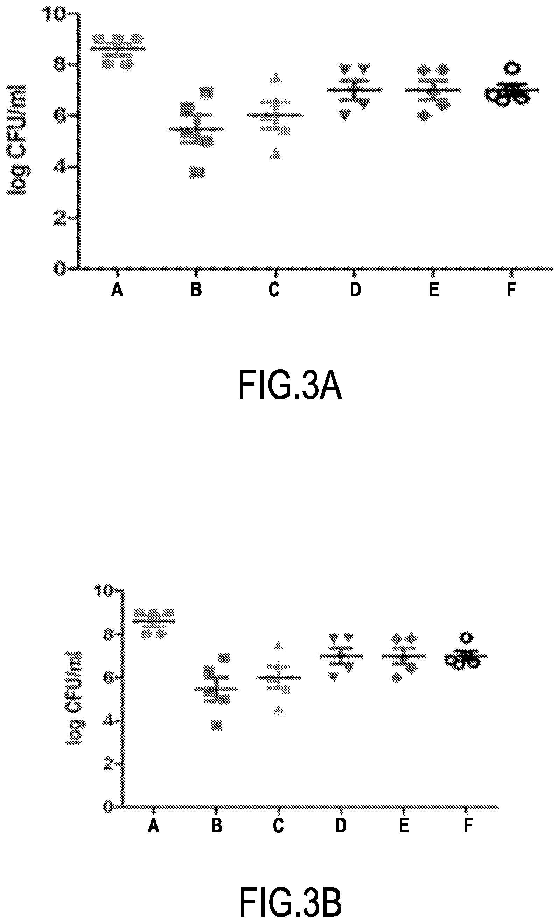

- les figures 3A et 3B illustrent les résultats de quantité de bactéries détectées en aérobiose à 48 heures (figure 3A) et en aérobiose après 6 semaines de conservation du lyophilisat à 4°C (figure 3B), par le log CFU/mL, sur des échantillons de selles avec les mêmes 5 conditions de milieux A, B, C, D, E et F ;

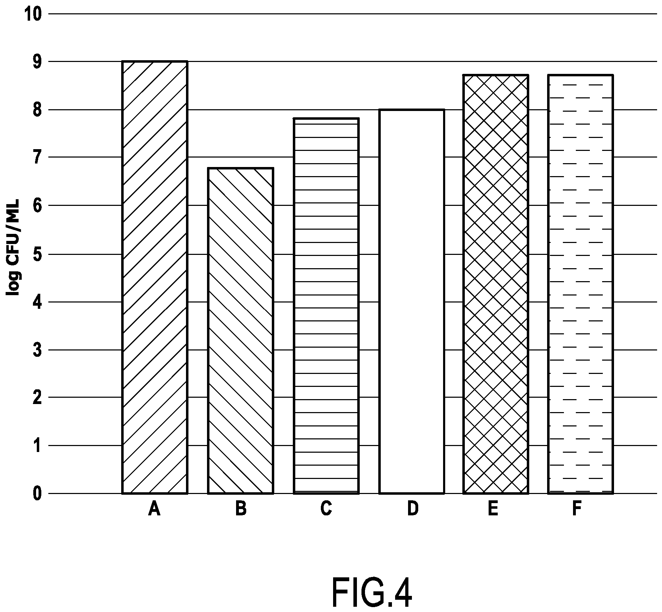

- la figure 4 représente des graphes illustrent les résultats de quantité de bactéries vivantes détectées dans un échantillon après lyophilisation d'un cocktail de 11 bactéries anaérobies par le log CFU/mL avec les mêmes conditions de milieu de conservation A,B, C ,D, E et F ;

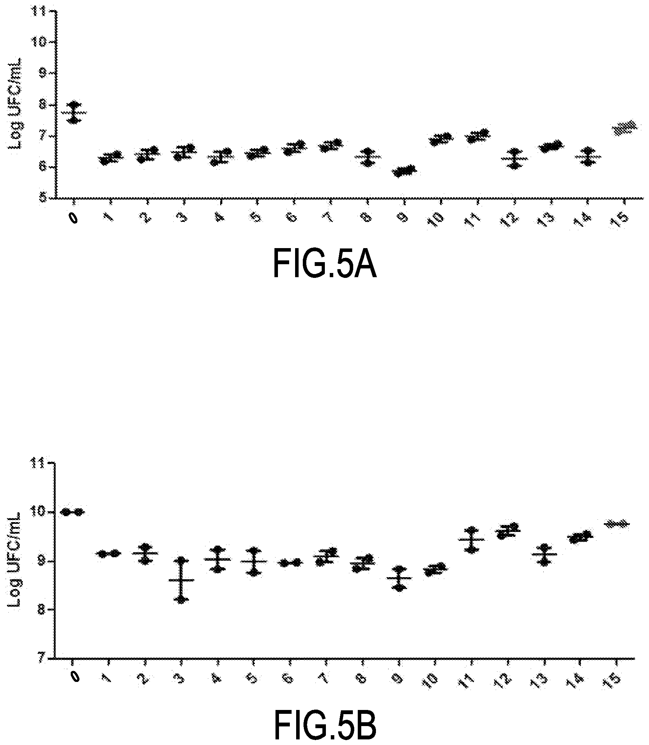

- les figures 5A et 5B illustrent les résultats de différences de quantité de bactéries vivantes détectées par les différences de nombres de log de CFU de bactéries aérobies (figure 5A) et bactéries anaérobies (figure 5B) sur des échantillons de selles avec différents agents protecteurs n°1 à 15 dans le milieu de conservation ;

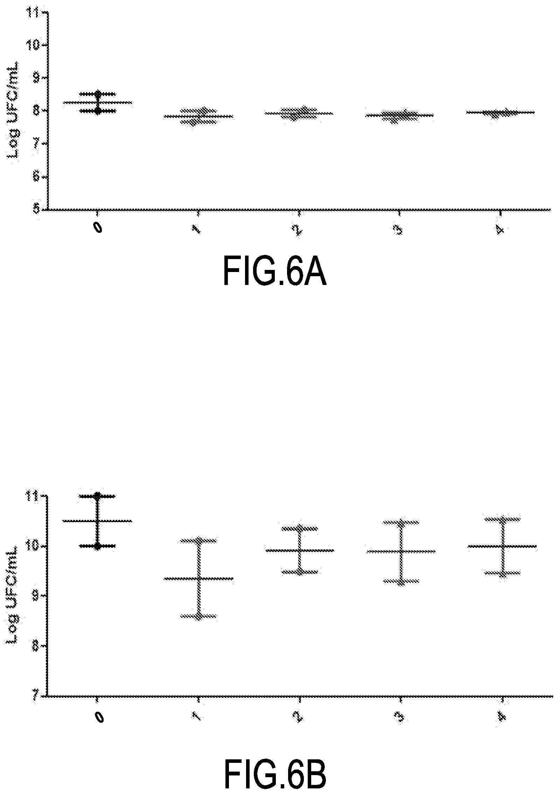

- les figures 6A et 6B illustrent les résultats comparatifs de différences de quantité de bactéries vivantes détectées par les différences de nombres de log de CFU de bactéries aérobies (figure 6A) et bactéries anaérobies (figure 6B) sur des échantillons de selles dans 4 conditions 1 à 4 avec différents agents protecteurs n°14 et 15 selon deux conditions différentes de congélation avant lyophilisation.

Sur les figures 5A-5B et 6A-6B, les cas « 0 » en abscisse correspondent aux résultats sur des selles fraîches.

### A) MATERIELS ET METHODES

### 1-Échantillons bactériens testés

Des échantillons de fèces ont été collectés à partir de donneurs sains, c'est-à-dire d'individus ne souffrant pas d'infection, n'ayant pas pris d'antibiotique le mois précédent l'échantillonnage, et ne souffrant pas de pathologie gastro-intestinale connue.

En parallèle, 29 souches bactériennes de la flore digestive ont été sélectionnées issues de la collection internationale de microorganismes CSUR (Collection de Souches de l'Unité des Rickettsies) de l'URMITE (WDCM 875) (Tableau 1 ci-après).

Les souches de bactéries sont stockées sous une forme adaptée à une congélation ultérieure dans des de tubes contenant dan bouillon de congélation contenant du glycérol des perles poreuses appelées cryobilles.

**Tableau 1: liste des 29 Souches bactériennes de références**

| **Bactérie** | **Atmosphère** | **N° CSUR** |
|---|---|---|
| *Bacteroides nordii* | Anaérobie | P1040 |
| *Propionibacterium avidum* | Anaérobie | P872 |
| *Clostridum irregular* | Anaérobie | P1913 |
| *Clostridum massilioamazoniensis* | Anaérobie | P1360 |
| *Clostridum butyricum* | Anaérobie | P607 |
| *Clostridum beijerinckii* | Anaérobie | P883 |
| *Bacteroides thetaiotaomicron* | Anaérobie | P766 |
| *Propionibacterium acnés* | Anaérobie | P742 |
| *Finegoldia magna* | Anaérobie | P588 |
| *Haemophilus influenzae* | Anaérobie | P1075 |
| *Bacteroides fragilis* | Anaérobie | P863 |
| *Staphylococcus aureus* | Aérobie | *P749* |
| *Enterobacter aerogenes* | Aérobie | P989 |
| *Escherichia coli* | Aérobie | P729 |
| *Klebsiella oxytoca* | Aérobie | P992 |
| *Streptococcus agalactiae* | Aérobie | P1030 |
| *Serratia marcescens* | Aérobie | P833 |
| *Enterococcus faecalis* | Aérobie | P720 |
| *Proteus mirabilis* | Aérobie | P2049 |
| *Pseudomonas aeruginosa* | Aérobie | P211 |
| *Streptococcus mitis* | Aérobie | P1123 |
| *Staphylococcus epidermidis* | Aérobie | P748 |
| *Morganella morganii* | Aérobie | P752 |
| *Citrobacter freundii* | Aérobie | P731 |
| *Enterobacter cloacae* | Aérobie | P713 |
| *Bacillus circulans* | Aérobie | P655 |
| *Streptococcus pneumoniae* | Aérobie | P825 |
| *Staphylococcus hominis* | Aérobie | P842 |
| *Acinetobacter baumanii* | Aérobie | P981 |

### 2-Protocole expérimental de congélation/lyophilisation

50 g. de selles dans 150cm3 dans un milieu liquide aqueux de conservation grâce à un mixeur (appareil BOSCH Ultracompact 400W) pendant 10 minutes. Cet échantillon est ensuite tamisé en utilisant un tamis alimentaires dédié (avec des pores de 2mm) afin d'éliminer les débris alimentaires et le filtrat est récolté dans des fioles en verre contenant du milieu de conservation (2mL) (shell vial type, REF 223683, Wheaton Millville, New Jersey, USA).

Les échantillons de souches bactériennes sont pareillement stockés dans des fioles en verre contenant du milieu de conservation (2mL) (shell vial type, REF 223683, Wheaton Millville, New Jersey, USA). Les échantillons ont ensuite été congelés soit pendant 5à -80°C soit dans l'azote liquide pendant 30sec à 2 min., puis déposés dans un lyophilisateur DELTA 1-24 LSC (Martin Christ Gefriertrocknungsanlagen GmbH, DE) pendant 36h, à une température de 0°C, et à une pression réduite de 0,63 mbar. Les lyophilisats obtenus ont été conservés à température ambiante.

### 3-Protocole d'études de viabilité

### 3.1 Dénombrement

La viabilité des microorganismes a été estimée par la méthode des Unités Formant Colonies (UFC) après dilutions décimales et étalement des échantillons de selles et échantillons de souches bactériennes testées sur des géloses au sang Columbia agar COS (BioMerieux, Craponne France). La dilution est exprimée en UFC/mL. Pour chaque dilution, trois boites de Pétri sont ensemencées et les boîtes sont incubées à 37°C pendant 48 heures dans deux conditions: aérobie et anaérobie (enceintes anaérobie ou générateurs d'anaérobie).

Ce travail a été réalisé à partir des:
- Echantillons bactériens frais et échantillons de selles fraîches, et
- Des lyophilisats réhydratés dans du tampon diphosphate sodique (PBS) à 25°C.

Les résultats obtenus à partir de selles fraîches (30 de l'émission) sont utilisés comme référence et comparés à ceux obtenus à partir du même échantillon préalablement congelé dans différents milieux de conservation puis le cas échéant lyophilisé.

On a évalué le nombre total de microorganismes viables après mise en culture en aérobiose et en anaérobiose sur boites de pétri, avant et après lyophilisation. La perte en microorganismes viable par rapport à la selle fraîche est exprimée en différence logarithmique de CFU perdues après les différents protocoles de congélation utilisés tout au long de l'expérimentation.

### 3.2 Identification des bactéries

Afin d'identifier les bactéries ayant survécu au processus de lyophilisation, les colonies ainsi cultivées sont identifiées de façon exhaustive par spectrométrie de masse MALDI-TOF à l'aide d'un spectromètre de masse MICROFLEX (Bruker Daltonics). Chaque dépôt est recouvert avec 2 ml de solution de matrice (acide α-saturé cyano-4-hydroxyciannamic dans 50% d'acétonitrile et d'acide trifluoroacetique 2,5%) comme décrit dans la littérature (ref.1). Cette analyse est effectuée en utilisant selon les recommandations du fabricant. Les spectres ainsi générés sont comparés avec la base de données de la base Bruker conjuguée à la base spécifique du laboratoire de l'hôpital de La Timone. Un isolat est considéré correctement identifié par MALDI-TOF si son score d'identification est supérieur à 1,9. Pour les colonies non identifiées, après 3 passages au MALDI-TOF, un séquençage de l'ARN 16S est réalisé (ref.2).

(ref.1) : Seng P, Drancourt M, Gouriet F, La Scola B, Fournier P-E, Rolain JM, et al. Ongoing revolution in bacteriology: routine identification of bacteria by matrix-assisted laser desorption ionization time-of-flight mass spectrometry. Clin Infect Dis Off Publ Infect Dis Soc Am. 2009 Aug 15;49(4):543-51.

(ref.2) :Morel AS, Dubourg G, Edouard S, Prudent E, Gouriet F, Casalta J et al. 2014. Complementarity between targeted real-time spécifie PCR and conventional broad-range 16S rDNA PCR in the syndrome-driven diagnosis of infectious diseases. Eur J Clin Microbiol Infect Dis 34: 561-570.

### 4) Milieux de conservation

Plusieurs milieux aqueux de conservation de pré-lyophilisation ont été testés permet d'augmenter la survie des micro-organismes au cours du séchage. Différentes combinaisons d'agents protecteurs ont été utilisées (Tableau 2 ci-après). Les échantillons dilués dans le milieu de conservation sont alors congelés durant 12H, puis lyophilisés.

Diverses combinaisons d'agents protecteurs suivantes ont été testées :
- glycérol seul,
- antioxydants seuls,
- lait seul,
- des combinaisons binaires : sucre + antioxydants, lait + sucre, lait + antioxydants, antioxydants + glycérol, sucre + glycérol,
- la combinaison ternaire : lait+ antioxydants + sucre.

Après différents essais comparatifs, les composés antioxydants les plus efficaces retenus comprenaient la combinaison ternaire suivante :
- 0,4g/L d'acide urique (*Sigma*-Aldrich,Lyon, France),
- 1g /Ld'acide ascorbique (Inresa Medical, Bartenheim, France),
- 0,1g /L de L-Glutathion (*Sigma*-Aldrich,Lyon, France),

Les agents protecteurs ont été mis en oeuvre dans de l'eau saline ou de l'eau saline + tampon diphosphate sodique PBS (voir tableau 3 ci-après).

L'eau saline comprenait :
- 0.3/0.6g KOH (BDH LABORATORY SUPPLIES, Poole, Royaume-Uni),
- 0.1g CaCl2 (Inresa Medical, Bartenheim, France),
- 0.1g MgCl2 (Inresa Medical, Bartenheim, France)

Le lait testé était du lait écrémé ou du lait entier en poudre. Les meilleurs résultats étaient obtenus avec du Lait entier en poudre (Régilait, Lyon, France) à une concentration pondérale de 10%.

Différents sucres ont été testés (lactose, glucose, tréhalose et saccharose) à différents concentrations. Les meilleurs résultats ont été obtenus avec du saccharose (aussi dénommé ci-après « sucrose ») et/ou tréhalose (Inresa Medical, Bartenheim, France) à une concentration en poids de 5 à 10%.

**Tableau 2: composition des milieux de conservation testés**

| **Milieu de conservation** | **Composition** |
|---|---|
| 1 | 150 mL eau saline |
| 2 | 150 mL PBS + |
| 3 | 150 mL PBS + antioxydants |
| 4 | 150 mL PBS + sucre |
| 5 | 150 mL PBS+ antioxydants + glycérol |
| 6 | 150 mL PBS+ sucre + antioxydants |
| 7 | 150 mL PBS + glycérol + sucre |
| 8 | 150 mL eau saline + Lait |
| 9 | 150 mL PBS + antioxydants + Lait |
| 10 | 150 mL PBS + sucre + Lait |
| 11 | 150 mL PBS + antioxydants + Lait + sucre |

La composition du milieu PBS était comme indiqué au tableau 3 ci-après.

**Tableau 3 : Milieu de conservation**

| | Concentration dans 1 litre (pH 7,3 ± 0,2) |
|---|---|
| KCl | 0.2g |
| CaCl2 | 0.1g |
| MgCl2 | 0.1g |
| KH2PO4 | 0.2g |
| Na2HPO4 | 1.15g |
| NaCl | 3g |
| KOH | 0.6g |

### B) RÉSULTATS ET DISCUSSION

On a évalué :
i) l'influence de la méthode de congélation, à savoir congélation à -80°C comparée à azote liquide (-196°C) avant la lyophilisation et
ii) l'impact des agents protecteurs dans le milieu de conservation.
1) Impact de la congélation

Tant pour les échantillons de bactéries pris séparément que pour les échantillons de selles, les résultats comparatifs quel que soit le milieu de conservation sont toujours meilleurs avec une congélation rapide dans l'azote liquide puis lyophilisation en comparaison à une congélation à -80°C pendant 12 H puis lyophilisation. La congélation rapide dans l'azote liquide puis lyophilisation permet une perte de CFU moins importante.

Les Figures 1A et 1B illustrent les nombres de log de CFU de bactéries aérobies (figure 1A) et respectivement bactéries anaérobies (figure 1B) perdus après les différents protocoles de congélation utilisés sur 6 échantillons de selles sans agents protecteurs dans le milieu de conservation :
- P1= congélation à -80°C pendant 12 H puis lyophilisation,
- P2= congélation rapide dans l'azote liquide puis lyophilisation,
- P3= congélation sans lyophilisation (selles fraîches).

Les figures 1A-1B illustrent que la préservation de la viabilité bactérienne après lyophilisation sans agents protecteurs demeure cependant significativement moins efficace qu'après congélation à -80°C durant 48H sans lyophilisation.

En revanche, la congélation rapide dans l'azote (-196°C) permet d'augmenter le taux de survie des dites bactéries, notamment du microbiote de selles humaines d'un facteur 100 (2 log) au moins par rapport à une congélation lente à -80°C avant lyophilisation.

### 2) Impact des agents protecteurs testés.

La présence de glycérol dans le milieu de conservation n'a pas d'impact positif sur la viabilité.

La présence d'antioxydants et de sucre seuls ou en combinaison binaires présente un impact positif mais qui ne permet pas d'améliorer la viabilité par rapport aux échantillons congelés sans lyophilisation.

En revanche, tant pour les échantillons de bactéries prises séparément que pour les échantillons des selles c'est à dire contenant tout le microbiote de la flore intestinale, il apparaît globalement une synergie pour le milieu de conservation comprenant du lait et un milieu de composition ternaire d'agents protecteurs comprenant : lait + antioxydants+ saccharose et/ou tréhalose qui permet d'atteindre un niveau de viabilité supérieur ou égal à celui avec un milieu de conservation avec du Lait seul et avec celui pour les selles congelées sans lyophilisation.

Les figures 2A et 2B illustrent les résultats de quantité de bactéries détectées mis en culture en anaérobiose après 48 heures de conservation du lyophilisat à 4°C (figure 2A) et en anaérobiose après 6 semaines de conservation du lyophilisat à 4°C (figure 2B) sur 6 échantillons de selles avec 5 conditions de milieu différentes: A= échantillon frais dans eau saline (ici selles fraîches), B= lyophilisat dans milieu PBS, C= Lyophilisat avec milieu PBS contenant des antioxydants seuls, D= Lyophilisat avec milieu PBS contenant du lait en poudre seul, E= Lyophilisat avec milieu PBS contenant antioxydants + saccharose + lait en poudre, F= congélation de l'échantillon frais dans milieu PBS sans agent protecteur.

Les figures 3A et 3B illustrent les résultats de quantité de bactéries détectées par mise en culture en aérobiose après 48 heures de conservation du lyophilisat à 4°C (figure 3A) et en aérobiose après 6 semaines de conservation du lyophilisat à 4°C (figure 3B) sur 6 échantillons de selles avec les mêmes 5 conditions de milieux différentes A, B, C, D, E et F.

La congélation à l'azote s'étant révélée plus efficace dans le chapitre précédent, toutes les étapes de lyophilisation des figures 2A-2B et 3A-3B sont précédés d'une étape de congélation par azote liquide à - 196°C.

Les résultats des tableaux 3A et 3B suivant quantifiés en Log CFU/mL montrent que le milieu de conservation pour la technique de lyophilisation le plus performant pour les selles demeure également le meilleur pour la conservation des échantillons de bactéries non lactiques pris séparément tant pour les bactéries aérobies (figure 3A) qu'anaérobies (figure 3B).

**Tableau 3A : BACTERIES AEROBIES (LOG CFU/mL)**

| | A | B | C | D | E | F |
|---|---|---|---|---|---|---|
| *Staphylococcus aureus* | 9 | 6,949 | 4,808 | 8 | 9 | 8,347 |
| *Enterobacter aerogenes* | 9 | 8,453 | 5,155 | 8,155 | 8,779 | 8,55 |
| *Escherichia coli* | 10 | 5,255 | 7,779 | 8,261 | 9,588 | 8,477 |
| *Klebsiella oxytoca* | 9 | 5,398 | 4,699 | 7,125 | 7,949 | 6,398 |
| *Streptococcus agalactiae* | 9 | 7 | 8,097 | 8,477 | 8 | 8,301 |
| *Serratia marcescens* | 9 | 4,431 | 5,574 | 8,096 | 9 | 8,176 |
| *Proteus mirabilis* | 9 | 8,477 | 7,699 | 9 | 9 | 8 |
| *Staphylococcus epidermidis* | 9 | 6 | 7,398 | 8,041 | 9 | 8 |
| *Bacillus circulans* | 8 | 6,602 | 7,301 | 7,301 | 7,7 | 7 |
| *Streptococcus pneumoniae* | 8,301 | 6,096 | 7,846 | 8,301 | 8,778 | 8,738 |
| *Staphylococcus hominis* | 9 | 8 | 8,398 | 8,812 | 8,853 | 8,787 |
| *Acinetobacter baumanii* | 9 | 7,289 | 8,041 | 8 | 8,544 | 8 |

**Tableau 3B**

| Anaérobies (Log CFU/mL) | | | | | | |
|---|---|---|---|---|---|---|
| | A | B | C | D | E | F |
| *Bacteroides nordii* | 8 | 0 | 5 | 4,699 | 7 | 6,633 |
| *Propionibacterium avidum* | 10 | 7,889 | 8,977 | 9,698 | 9,176 | 9,477 |
| *Clostridum irregular* | 8 | 6,875 | 6,368 | 5,301 | 7,65 | 7,699 |
| *Clostridum massilioamazoniensis* | 6 | 3,8 | 4,202 | 5,875 | 5,414 | 5 |
| *Clostridum butyricum* | 9 | 7,699 | 7,477 | 8,621 | 8,155 | 7 |
| *Clostridum beijerinckii* | 9 | 7,588 | 8,602 | 8,085 | 8,7 | 8,199 |
| *Bacteroides thetaiotaomicron* | 9 | 0 | 0 | 3 | 4 | 5,7 |
| *Propionibacterium acnes* | 9 | 6,796 | 8,301 | 8,041 | 8,544 | 8,729 |
| *Finegoldia magna* | 9 | 6,103 | 7,666 | 8,155 | 8,653 | 8,478 |
| *Bacteroides fragilis* | 9 | 2 | 4 | 5,951 | 6,5 | 7,801 |

Enfin, les graphes de la figure 4 illustrent que le milieu ternaire E (Lyophilisat avec antioxydants + saccharose + lait en poudre) précédemment décrit présente les meilleurs résultats pour la lyophilisation d'un cocktail de 11 bactéries anaérobies suivantes en comparaison aux mêmes autres conditions de milieu de conservation A,B, C ,D et F ci-dessus : *Bacteroides fragilis, Bacteroides nordii, Bacteroides thetaiotaomicron, Clostidium butyricum, Clostridium massilioamazoniensis, Clostridium beijerinckii, Clostridium irregular Finegoldia magna, Propionibacterium acnes, Propionibacterium avidum, Haemophilus influenza.*

Dans tous les cas on observe que l'on obtient avec les milieux D (Lait seul) ou de préférence E (lait+antioxydants+sucre) des lyophilisats comprenant :
- au moins 10⁸ CFU/mL bactéries de chacune des catégories des bactéries aérobies et respectivement bactéries anaérobies mises en culture après 48h de conservation à 4°C, et
- au moins 10⁸ CFU/mL bactéries de chacune des catégories des bactéries aérobies et respectivement bactéries anaérobies mise en culture après 6 semaines de conservation à 4°C, et
- plus généralement, la congélation rapide dans l'azote (-196°C) permet d'augmenter le taux de survie des dites bactéries du microbiote intestinal d'un facteur 100 au moins par rapport à une congélation lente à -80°C avant lyophilisation.

Sur les figures 5A et 5B, on a comparé les résultats sur deux selles issues de deux donneurs sains des 15 milieux de conservation n°1 à 15 suivants avec les agents protecteurs suivants :
- 1-: sucrose 10%
- 2-: tréhalose 5%
- 3-: lait 10%
- 4-: antioxydants
- 5-: lait 10% + antioxydants
- 6-: lait 10%+ tréhalose 5%
- 7-: lait 10%+ sucrose 10%
- 8-: tréhalose 5% + sucrose 10%
- 9-: tréhalose 5% + antioxydants
- 10-: sucrose 10%+ antioxydants
- 11-: sucrose 10%+ tréhalose5% + antioxydants
- 12-: lait 10% + tréhalose 5% + sucrose10%
- 13-: lait 10% + tréhalose 5%+ antioxydants
- 14-: lait 10%+ sucrose10%+antioxydants
- 15-: lait 10%+sucrose 10% + tréhalose 5 % +antioxydants

La congélation était effectué à -80°C pendant 5 heures.

La Lyophilisation était effectuée dans le lyophilisateur DELTA 1-24 LSC-CHRIST à pression de 0.63 mbar, et température du plateau à 0 °C pendant 36 heures suivi de 6h à +30 °C (selon la méthode de Staley et al. Am J Gastro 2017).

Les résultats de viabilité sont rapportés dans les figures 5A et 5B et tableau 4 suivant (valeurs moyennes).

**Tableau 4 : Pourcentage de viabilité bactérienne après lyophilisation**

| | Selle 1 | | Selle 2 | |
|---|---|---|---|---|
| | **Anaérobies (%viabilité)** | **Aérobies (% viabilité)** | **Anaérobies (%viabilité)** | **Aérobies (%viabilité)** |
| **1-Sucrose 10%** | 91,18 | 87,16 | 91.43 | 78,37 |
| **2-Tréhalose 5%** | 93,14 | 88,54 | 90,12 | 78,84 |
| **3-Lait 10%** | 90,61 | 89,29 | 83 | 79,46 |
| **4-Antioxydants** | 86,16 | 87,81 | 92,48 | 77,86 |
| **5-Lait 10%+ antioxydants** | 92,56 | 88,52 | 87,88 | 79,63 |
| **6-Lait 10% + tréhalose 5%** | 89,23 | 90,97 | 89,74 | 81,27 |
| **7-Lait 10% + sucrose 10%** | 92,72 | 91,60 | 89,76 | 82,73 |
| **8-Tréhalose 5% +sucrose 10%** | 92,03 | 87,65 | 88,41 | 77,43 |
| **9-Tréhalose 5% + antioxydants** | 91,95 | 83,11 | 84,87 | 73,28 |
| **10-Sucrose 10% + antioxydants** | 90,38 | 93,73 | 87,95 | 85,38 |
| **11-Sucrose 10% + tréhalose 5%+ antioxydants** | 91,35 | 95,20 | 96,07 | 86,47 |
| **12-Lait 10% +tréhalose 5% + sucrose10%** | 93,13 | 87,65 | 96,95 | 76,82 |
| **13-Lait 10%+tréhalose 5% +antioxydants** | 89,95 | 90,90 | 92,46 | 82,82 |
| **14-Lait 10%+sucrose10% +antioxydants** | 92,10 | 88,59 | 95,32 | 77,47 |
| **15-Lait 10%+sucrose10%+ tréhalose 5 % +antioxydants** | **97,34** | **98,34** | **97,37** | **89,31** |

**Tableau 5: composition des milieux de conservation testés**

| Solution 1 | | | | | | | |
|---|---|---|---|---|---|---|---|
| S1 = sucrose 10% | | | Concentration dans 1 litre de milieu (pH 7,3 ± 0,2) | | Référence | | |
| PBS | | | 1L | | Réf 14190-094, Life Technologies,Paiseley, Royaume-Uni | | |
| sucrose 10% | | | 100g | | Réf 57501-1,Inresa Medical, Bartenheim, France | | |

| Solution 2 | | | | | | | |
|---|---|---|---|---|---|---|---|
| S2 = tréhalose 5% | | | Concentration dans 1 litre de milieu (pH 7,3 ± 0,2) | Référence | | | |
| PBS | | | 1L | Réf 14190-094, Life Technologies,Paiseley, Royaume-Uni | | | |
| Tréhalose 5% | | | 50g | Réf 194756,MP biomedicals LLC illkirch ,France | | | |

| Solution 3 | | | | | | | |
|---|---|---|---|---|---|---|---|
| S3= Lait 10% | | Concentration dans 1 litre de milieu (pH 7,3 ± 0,2) | | | Référence | | |
| PBS | | 1L | | | Réf 14190-094, Life Technologies,Paiseley, Royaume-Uni | | |
| Lait demi écrémé 10% | | 100g | | | Régilait, Lyon, France | | |

| Solution 4 | | | | | | | |
|---|---|---|---|---|---|---|---|
| S4 = antioxydants | | Concentration dans 1 litre de milieu (pH 7,3 ± 0,2) | | Référence | | | |
| PBS | | 1L | | Réf 14190-094, Life Technologies,Paiseley, Royaume-Uni | | | |
| CaCl₂ | | 0.1g | | Réf 10035,Inresa Medical, Bartenheim, France | | | |
| MgCl₂ | | 0.1g | | Réf 77911,Inresa Medical, Bartenheim, France | | | |
| KOH | | 0.3/0.6g | | Réf 102105W,BDH LABORATORY SUPPLIES, Poole, Royaume-Uni | | | |
| Acide ascorbique | | 1g | | Réf 50817, Inresa Medical, Bartenheim, France | | | |
| Acide urique | | 0.4g | | Réf U2625,Sigma-Aldrich,Lyon, France | | | |
| Glutathion | | 0.1g | | Réf G4251,Sigma-Aldrich,Lyon, France | | | |

| Solution 5 | | | | | | | |
|---|---|---|---|---|---|---|---|
| S5= Lait 10% +antioxydants | | | Concentration dans 1 litre de milieu (pH 7,3 ± 0,2) | | | Référence | |
| PBS | | | 1L | | | Réf 14190-094, Life Technologies,Paiseley, Royaume-Uni | |
| Lait demi écrémé 10% | | | 100g | | | Régilait, Lyon, France | |
| CaCl₂ | | | 0.1g | | | Réf 10035,Inresa Medical, Bartenheim, France | |
| MgCl₂ | | | 0.1g | | | Réf 77911,Inresa Medical, Bartenheim, France | |
| KOH | | | 0.3/0.6g | | | Réf 102105W,BDH LABORATORY SUPPLIES, Poole, Royaume-Uni | |
| Acide ascorbique | | | 1g | | | Réf 50817,Inresa Medical, Bartenheim, France | |
| Acide urique | | | 0.4g | | | Réf U2625,Sigma-Aldrich,Lyon, France | |
| Glutathion | | | 0.1g | | | Réf G4251,Sigma-Aldrich,Lyon, France | |

| Solution 6 | | | | | | | |
|---|---|---|---|---|---|---|---|
| S6 = Lait10%+ tréhalose 5% | | | Concentration dans 1 litre de milieu (pH 7,3 ± 0,2) | Référence | | | |
| PBS | | | 1L | Réf 14190-094, Life Technologies,Paiseley, Royaume-Uni | | | |
| Lait demi écrémé 10% | | | 100q | Régilait, Lyon, France | | | |
| Tréhalose 5% | | | 50g | Réf 194756,MP biomedicals LLC illkirch ,France | | | |

| Solution 7 | | | | | | | |
|---|---|---|---|---|---|---|---|
| S7= Lait10%+sucrose 10% | | | Concentration dans 1 litre de milieu (pH 7,3 ± 0,2) | | Référence | | |
| PBS | | | 1L | | Réf 14190-094, Life Technologies,Paiseley, Royaume-Uni | | |
| Lait demi écrémé 10% | | | 100g | | Régilait, Lyon, France | | |
| sucrose10% | | | 100g | | Réf 57501-1,Inresa Medical, Bartenheim, France | | |

| Solution 8 | | | | | | | |
|---|---|---|---|---|---|---|---|
| S8= sucrose10%+ tréhalose5% | | | | Concentration dans 1 litre milieu (pH ± 0,2) | Référence de 7,3 | | |
| PBS | | | | 1L | Réf 14190-094, Life Technologies,Paiseley, Royaume-Uni | | |
| sucrose10% | | | | 100g | Réf 57501-1,Inresa Medical, Bartenheim, France | | |
| Tréhalose 5% | | | | 50g | Réf 194756,MP biomedicals LLC illkirch ,France | | |

| Solution 9 | | | | | | | |
|---|---|---|---|---|---|---|---|
| S9= Trehalose5%+antioxydants | | | | Concentration Référence dans 1 litre de milieu (pH 7,3 ± 0,2) | | | |
| PBS | | | | 1L | Réf 14190-094, Life Technologies,Paiseley, Royaume-Uni | | |
| Tréhalose 5% | | | | 50g | Réf 194756,MP biomedicals LLC illkirch ,France | | |
| CaCl₂ | | | | 0.1g | Réf 10035,Inresa Medical, Bartenheim, France | | |
| MgCl₂ | | | | 0.1g | Réf 77911,Inresa Medical, Bartenheim, France | | |
| KOH | | | | 0.3/0.6g | Réf 102105W,BDH LABORATORY SUPPLIES, Poole, Royaume-Uni | | |
| Acide ascorbique | | | | 1g | Réf 50817,Inresa Medical, Bartenheim, France | | |
| Acide urique | | | | 0.4g | Réf U2625,Sigma-Aldrich,Lyon, France | | |
| Glutathion | | | | 0.1g | Réf G4251,Sigma-Aldrich,Lyon, France | | |

| Solution 10 | | | | | | | |
|---|---|---|---|---|---|---|---|
| S10= sucrose 10%+antioxydants | | | Concentration 1 litre de milieu (pH 7,3 ± 0,2) | | dans Référence | | |
| PBS | | | 1L | | Réf 14190-094, Life Technologies,Paiseley, Royaume-Uni | | |
| Sucrose 10% | | | 100g | | Réf 57501-1,Inresa Medical, Bartenheim, France | | |
| CaCl₂ | | | 0.1g | | Réf 10035,Inresa Medical, Bartenheim, France | | |
| MgCl₂ | | | 0.1g | | Réf 77911,Inresa Medical, Bartenheim, France | | |
| KOH | | | 0.3/0.6g | | Réf 102105W,BDH LABORATORY SUPPLIES, Poole, Royaume-Uni | | |
| Acide ascorbique | | | 1g | | Réf 50817,Inresa Medical, Bartenheim, France | | |
| Acide urique | | | 0.4g | | Réf U2625,Sigma-Aldrich,Lyon, France | | |
| Glutathion | | | 0.1g | | Réf G4251,Sigma-Aldrich,Lyon, France | | |

| Solution 11 | | | | | | | |
|---|---|---|---|---|---|---|---|
| S11= sucrose 10%+ tréhalose 5% +antioxydants | Concentration dans 1 litre de milieu (pH 7,3 ± 0,2) | | | | Référence | | |
| PBS | 1L | | | | Réf 14190-094, Life Technologies,Paiseley, Royaume-Uni | | |
| Sucrose 10% | 100g | | | | Réf 57501-1,Inresa Medical, Bartenheim, France | | |
| Tréhalose 5% | 50g | | | | Réf 194756,MP biomedicals LLC illkirch ,France | | |
| CaCl₂ | 0.1g | | | | Réf 10035,Inresa Medical, Bartenheim, France | | |
| MgCl₂ | 0.1g | | | | Réf 77911,Inresa Medical, Bartenheim, France | | |
| KOH | 0.3/0.6g | | | | Réf 102105W,BDH LABORATORY SUPPLIES, Poole, Royaume-Uni | | |
| Acide ascorbique | 1g | | | | Réf 50817,Inresa Medical, Bartenheim, France | | |
| Acide urique | 0.4g | | | | Réf U2625,Sigma-Aldrich,Lyon, France | | |
| Glutathion | 0.1g | | | | Réf G4251,Sigma-Aldrich,Lyon, France | | |

| Solution 12 | | | | | | | |
|---|---|---|---|---|---|---|---|
| S12= Lait10%+ tréhalose 5%+sucrose 10% | | | | Concentration dans 1 litre de milieu (pH 7,3 ± 0,2) | | | Référence |
| PBS | | | | 1L | | | Réf 14190-094, Life Technologies,Paiseley, Royaume-Uni |
| Lait demi écrémé 10% | | | | 100g | | | Régilait, Lyon, France |
| Sucrose 10% | | | | 100g | | | Réf 57501-1,Inresa Medical, Bartenheim, France |
| Tréhalose 5% | | | | 50g | | | Réf 194756,MP biomedicals LLC illkirch ,France |

| Solution 13 | | | | | | | |
|---|---|---|---|---|---|---|---|
| S13=Lait 10%+5% tréhalose +antioxydants | | | Concentration dans 1 litre de milieu (pH 7,3 ± 0,2) | | Référence | | |
| PBS | | | 1L | | Réf 14190-094, Life Technologies,Paiseley, Royaume-Uni | | |
| Lait demi écrémé 10% | | | 100g | | Régilait, Lyon, France | | |
| Tréhalose 5% | | | 50g | | Réf 194756,MP biomedicals LLC illkirch ,France | | |
| CaCl₂ | | | 0.1g | | Réf 10035,Inresa Medical, Bartenheim, France | | |
| MgCl₂ | | | 0.1g | | Réf 77911,Inresa Medical, Bartenheim, France | | |
| KOH | | | 0.3/0.6g | | Réf 102105W,BDH LABORATORY SUPPLIES, Poole, Royaume-Uni | | |
| Acide ascorbique | | | 1g | | Réf 50817,Inresa Medical, Bartenheim, France | | |
| Acide urique | | | 0.4g | | Réf U2625,Sigma-Aldrich,Lyon, France | | |
| Glutathion | | | 0.1g | | Réf G4251,Sigma-Aldrich,Lyon, France | | |

| Solution 14 | | | | | | | |
|---|---|---|---|---|---|---|---|
| S14=Lait 10%+ sucrose 10%+ +antioxydants | | Concentration dans 1 litre de milieu (pH 7,3 ± 0,2) | | | | Référence | |
| PBS | | 1L | | | | Réf 14190-094, Life Technologies,Paiseley, Royaume-Uni | |
| Lait demi écrémé 10% | | 100g | | | | Régilait, Lyon, France | |
| Sucrose 10% | | | | 100g | | | Réf 57501-1,Inresa Medical, Bartenheim, France |
| CaCl₂ | | | | 0.1g | | | Réf 10035,Inresa Medical, Bartenheim, France |
| MgCl₂ | | | | 0.1g | | | Réf 77911,Inresa Medical, Bartenheim, France |
| KOH | | | | 0.3/0.6g | | | Réf 102105W,BDH LABORATORY SUPPLIES, Poole, Royaume-Uni |
| Acide ascorbique | | | | 1g | | | Réf 50817,Inresa Medical, Bartenheim, France |
| Acide urique | | | | 0.4g | | | Réf U2625,Sigma-Aldrich,Lyon, France |
| Glutathion | | | | 0.1g | | | Réf G4251,Sigma-Aldrich,Lyon, France |

| Solution 15 | | | | | | | |
|---|---|---|---|---|---|---|---|
| S15=Lait 10%+ sucrose10%+ tréhalose 5%+ +antioxydants | | | | | Concentration dans 1 litre de milieu (pH 7,3 ± 0,2) | Référence | |
| PBS | | | | | 1L | Réf 14190-094, Life Technologies,Paiseley, Royaume-Uni | |
| Lait demi écrémé 10% | | | | | 100q | Régilait, Lyon, France | |
| Sucrose 10% | | | | | 100q | Réf 57501-1,Inresa Medical, Bartenheim, France | |
| Tréhalose 5% | | | | | 50g | Réf 194756,MP biomedicals LLC illkirch ,France | |
| CaCl₂ | | | | | 0.1g | Réf 10035,Inresa Medical, Bartenheim, France | |
| MgCl₂ | | | | | 0.1g | Réf 77911,Inresa Medical, Bartenheim, France | |
| KOH | | | | | 0.3/0.6g | Réf 102105W,BDH LABORATORY SUPPLIES, Poole, Royaume-Uni | |
| Acide ascorbique | | | | | 1g | Réf 50817,Inresa Medical, Bartenheim, France | |
| Acide urique | | | | | 0.4g | Réf U2625,Sigma-Aldrich,Lyon, France | |
| Glutathion | | | | | 0.1g | Réf G4251,Sigma-Aldrich,Lyon, France | |

Les résultats de viabilité ont montré que la présence de chacun des agents cryoprotecteurs seuls (lait ; tréhalose ; sucrose) ou avec des antioxydants améliore fortement la survie des bactéries après lyophilisation. Cependant la conservation est maximale en présence des 3 agents cryoprotecteurs plus les antioxydants (milieu n°15). Le milieu n° 15 a permis une meilleure protection des bactéries après lyophilisation que ce soit en anaérobiose (~ 97%) ou en aérobiose (>90%).

Les figures 6A et 6B illustrent les résultats comparatifs de viabilité en fonction des conditions de conditions de congélation avant lyophilisation (étape de pré-lyophilisation). On a comparé les résultats sur deux selles issues de deux donneurs sains à partir de 2 milieux de lyophilisation différents et selon deux modes de congélation différents.

Les deux milieux testés comprenaient les agents protecteurs suivants :
1- Lait 10%+ sucrose10% + antioxydants
2- Lait 10% + sucrose 10% + tréhalose 5 % + antioxydants.

Les deux modes de congélations comprenaient :
- une congélation à -80°C pendant 5 heures, et
- une congélation rapide dans l'azote (-195,79 °C) pendant 1-2 min.

La lyophilisation subséquente était identique dans tous les cas à savoir à pression de 0.63 mbar, et température du plateau à 0 °C pendant 36 heures suivi de 6h à +30 °C dans le lyophilisateur DELTA 1-24 LSC-CHRIST.

Sur les figures 6A et 6B et le tableau 5(valeurs moyennes) sont rapportés les résultats de viabilité pour les 4 conditions n°1 à 4(en abscisses) suivantes :
- condition n°1= congélation à -80°C / lyophilisation milieu n°14 (Lait 10%+ sucrose10% +antioxydants),
- condition n°2= congélation à -80°C / lyophilisation milieu n° 15 (Lait 10%+ sucrose 10% + tréhalose 5 % +antioxydants),
- condition n°3= congélation dans l'azote / lyophilisation milieu 14 (- Lait 10%+ sucrose10% +antioxydants), et
- condition n°4= congélation dans l'azote / lyophilisation milieu 15 (Lait 10%+ sucrose 10% + tréhalose 5 % +antioxydants).

Tableau 6 : Pourcentage de viabilité bactérienne après lyophilisation.

| | % de viabilité | | Conditions | | | |
|---|---|---|---|---|---|---|
| | | **1** | | **2** | **3** | **4** |
| Selle3 | % Anaérobies | 86,20 | | **94,52** | 92,48 | **94,07** |
| | % Aérobies | 95,53 | | **97,56** | 96,89 | **98,92** |
| Selle4 | % Anaérobies | 91,27 | | **93,55** | 94,64 | **95,24** |
| | % Aérobies | 93,5 | | **94,03** | 92,69 | **92,91** |

Les résultats de cette étude ont montré qu'entre (a) la congélation à -80°C pendant 5 H puis lyophilisation et (b) la congélation rapide dans l'azote liquide puis lyophilisation directe, la congélation dans l'azote liquide permet une perte de CFU moins importante.

On a comparé les résultats de viabilité de bactéries aérobies et anaérobies sur deux selles issues de deux donneurs sains à partir d'un même milieu de lyophilisation (milieu n°15= Lait 10%+ sucrose 10% + tréhalose 5 % +antioxydants) congelé par congélation rapide dans l'azote (-195,79 °C) pendant 1-2 min. dans les deux cas mais avec deux conditions de lyophilisation différentes dans le Lyophilisateur DELTA 1-24 LSC-CHRIST. Les deux conditions de lyophilisation étaient :
1) pression de 0.63 mbar, et température du plateau à 0°C pendant 12 heures ; et
2) pression de 0.63 mbar et température du plateau à 0°C pendant 36 heures suivi de 6h à +30 °C (selon la méthode de Staley et al. Am J Gastro 2017).

Les résultats ne montrent pas de différence entre les deux méthodes de lyophilisation.

## Revendications

1. Procédé de conservation d'un échantillon d'un ensemble de bactéries aérobies et anaérobies autres que des bactéries lactiques, notamment des bactéries du microbiote intestinal, comprenant les étapes successives suivantes dans lesquelles :
a) on prépare un dit échantillon d'un ensemble de dites bactéries par dilution dans un milieu aqueux de conservation comprenant une combinaison d'agents protecteurs consistant en (a1) une combinaison de composés antioxydants choisis parmi de l'acide ascorbique, de l'acide urique et du glutathion, (a2) au moins 2 sucres différents consistant en du saccharose et du tréhalose, et (a3) des protéines de lait, et
b) on réalise une congélation dudit échantillon récupéré à l'étape a) dans de l'azote liquide à environ -196°C pendant pas plus de 2 minutes de préférence pendant 30 à 50 secondes, et
c) on réalise une lyophilisation du dit échantillon ainsi congelé pour obtenir un lyophilisat.

2. Procédé selon la revendication 1, **caractérisé en ce qu'**à l'étape a), on prépare un dit échantillon qui est un échantillon clinique de selles par dilution desdites selles dans un milieu aqueux de conservation, et on filtre la dispersion obtenue à pour en retirer au moins les débris de tailles supérieures à 2mm.

3. Procédé selon l'une des revendications 1 à 2, **caractérisé en ce que** les protéines de lait sont sous forme de lait entier, de préférence à une concentration pondérale dans le dit milieu de conservation d'au moins 5%, de préférence 10%.

4. Procédé selon l'une des revendications 1 à 3, **caractérisé en ce que** les dits agents protecteurs comprennent une combinaison de composés antioxydants consistant dans de l'acide ascorbique à une concentration de 1g/L, de l'acide urique à une concentration de 0,4g/L et du glutathion à une concentration de 0,1g/L.

5. Procédé selon l'une des revendications 1 à 4 **caractérisé en ce que** les dits agents protecteurs comprennent au moins un dit sucre à une concentration pondérale dans le dit milieu de conservation d'au moins 5%, de préférence 10%.

6. Procédé selon l'une des revendications 1 à 5, **caractérisé en ce que** le dit milieu de conservation est un milieu tampon à PH de 7 à 7.5 comprenant dans de l'eau les sels KCL, CaCl₂, MgCl₂,KH₂PO₄, Na₂HPO₄, NaCL et KOH ainsi qu'au moins un dit agent protecteur.

7. Procédé selon l'une des revendications 1 à 6, **caractérisé en ce que** le dit milieu de conservation comprend :
(a1) une combinaison de composés antioxydants consistant dans de l'acide ascorbique à une concentration de 1g/L, de l'acide urique à une concentration de 0,4g/L et du glutathion à une concentration de 0,1g/L, et
(a2) du saccharose à une concentration pondérale dans le dit milieu de conservation de 5%, et du tréhalose à une concentration pondérale dans le dit milieu de conservation de10%, et
(a3) des protéines de lait à une concentration pondérale dans le dit milieu de conservation de 10%.

8. Procédé selon l'une des revendications 1 à 7, **caractérisé en ce que** le dit échantillon comprend au moins les bactéries aérobies et bactéries anaérobies suivantes : *Staphylococcus aureus, Enterobacter aerogenes, Escherichia coli, Klebsiella oxytoca, Streptococcus agalactiae, Serratia marcescens, Proteus mirabilis, Staphylococcus epidermidis, Bacillus circulans, Streptococcus pneumoniae, Staphylococcus hominis et Acinetobacter baumanii.*

9. Procédé l'une des revendications 1 à 8, **caractérisé en ce que** le dit échantillon comprend au moins les bactéries anaérobies strictes suivantes : *Bacteroides fragilis, Bacteroides nordii, Bacteroides thetaiotaomicron, Clostidium butyricum, Clostridium massilioamazoniensis, Clostridium beijerinckii, Clostridium irregular Finegoldia magna, Propionibacterium acnes, Propionibacterium avidum, Haemophilus influenzae.*

10. Procédé selon l'une des revendications 1 à 9, **caractérisé en ce que** à l'étape c) l'échantillon congelé est déposé dans un lyophilisateur pendant au moins 12h à une température d'au moins 0°C et à une pression de vide poussé de pas plus de 1mbar.

11. Lyophilisat d'échantillon d'un ensemble de bactéries aérobies et anaérobies du microbiote intestinal autres que des bactéries lactiques, obtenu par un procédé selon l'une des revendications 1 à 10, **caractérisé en ce qu'**il comprend :
- au moins un agent protecteur consistant dans au moins la combinaison d'agents protecteurs consistant dans *(a1) une combinaison de composés antioxydants choisis parmi de l'acide ascorbique, de l'acide urique et du glutathion, a2) au moins 2 sucres différents consistant en du saccharose et du tréhalose, et a3) des protéines de lait,* et
- une quantité de bactéries vivantes, de préférence au nombre de :
- au moins 10⁸ CFU/mL bactéries de chacune des catégories des bactéries aérobies et respectivement bactéries anaérobies mises en culture après 48h de conservation à 4°C, et
- au moins 10⁸ CFU/mL bactéries de chacune des catégories des bactéries aérobies et respectivement bactéries anaérobies mise en culture après 6 semaines de conservation à 4°C.

12. Lyophilisat selon la revendication 11 **caractérisé en ce que** le dit échantillon est un échantillon clinique de selles humaines, les dits agents protecteurs consistant dans a1) une combinaison de 3 composés antioxydants consistant dans de l'acide ascorbique, de l'acide urique et du glutathion, et a2) au moins 2 sucres différents consistant dans du saccharose et du tréhalose, et a3) des protéines de lait.

## Patentansprüche

1. Verfahren zur Konservierung einer Probe einer Gruppe von aeroben und anaeroben Bakterien, die keine Milchsäurebakterien sind, insbesondere von Bakterien der Darmflora, umfassend die folgenden aufeinanderfolgenden Schritte, bei denen:
a) eine Probe einer Gruppe der Bakterien durch Verdünnen in einem wässrigen Konservierungsmedium bereitet wird, das eine Kombination von Schutzmitteln umfasst, die aus (a1) einer Kombination von antioxidativen Verbindungen, die aus Ascorbinsäure, Harnsäure und Glutathion gewählt sind, (a2) mindestens 2 verschiedenen Zuckern, die aus Saccharose und Trehalose bestehen, und (a3) Milchproteinen bestehen, und
b) ein Einfrieren der in Schritt a) gewonnenen Probe in flüssigem Stickstoff bei etwa -196 °C während nicht mehr als 2 Minuten, bevorzugt während 30 bis 50 Sekunden, durchgeführt wird und
c) eine Gefriertrocknung der so eingefrorenen Probe durchgeführt wird, um ein Lyophilisat zu erhalten.

2. Verfahren nach Anspruch 1, **dadurch gekennzeichnet, dass** im Schritt a) eine Probe, die eine klinische Stuhlprobe ist, durch Verdünnen des Stuhls in einem wässrigen Konservierungsmedium bereitet wird und die erhaltene Dispersion gefiltert wird, um mindestens die Stückchen mit Größen über 2 mm daraus zu entfernen.

3. Verfahren nach einem der Ansprüche 1 bis 2, **dadurch gekennzeichnet, dass** die Milchproteine in Form von Vollmilch vorliegen, bevorzugt in einer Gewichtskonzentration in dem Konservierungsmedium von mindestens 5 %, bevorzugt 10 %.

4. Verfahren nach einem der Ansprüche 1 bis 3, **dadurch gekennzeichnet, dass** die Schutzmittel eine Kombination von antioxidativen Verbindungen umfassen, die aus Ascorbinsäure in einer Konzentration von 1 g/l, Harnsäure in einer Konzentration von 0,4 g/l und Glutathion in einer Konzentration von 0,1 g/l bestehen.

5. Verfahren nach einem der Ansprüche 1 bis 4, **dadurch gekennzeichnet, dass** die Schutzmittel mindestens einen Zucker in einer Gewichtskonzentration in dem Konservierungsmedium von mindestens 5 %, bevorzugt 10 %, umfassen.

6. Verfahren nach einem der Ansprüche 1 bis 5, **dadurch gekennzeichnet, dass** das Konservierungsmedium ein Puffermedium mit einem pH-Wert von 7 bis 7,5 ist, das in Wasser die Salze KCl, CaCl₂, MgCl₂, KH₂PO₄, Na₂HPO₄, NaCl und KOH sowie mindestens ein Schutzmittel umfasst.

7. Verfahren nach einem der Ansprüche 1 bis 6, **dadurch gekennzeichnet, dass** das Konservierungsmedium umfasst:
(a1) eine Kombination von antioxidativen Verbindungen, die aus Ascorbinsäure in einer Konzentration von 1 g/l, Harnsäure in einer Konzentration von 0,4 g/l und Glutathion in einer Konzentration von 0,1 g/l bestehen, und
(a2) Saccharose in einer Gewichtskonzentration in dem Konservierungsmedium von 5 % und Trehalose in einer Gewichtskonzentration in dem Konservierungsmedium vom 10 % und
(a3) Milchproteine in einer Gewichtskonzentration in dem Konservierungsmedium von 10 %.

8. Verfahren nach einem der Ansprüche 1 bis 7, **dadurch gekennzeichnet, dass** die Probe mindestens die folgenden aeroben Bakterien und anaeroben Bakterien umfasst: *Staphylococcus aureus, Enterobacter aerogenes, Escherichia coli, Klebsiella oxytoca, Streptococcus agalactiae, Serratia marcescens, Proteus mirabilis, Staphylococcus epidermidis, Bacillus circulans, Streptococcus pneumoniae, Staphylococcus hominis und Acinetobacter baumanii.*

9. Verfahren nach einem der Ansprüche 1 bis 8, **dadurch gekennzeichnet, dass** die Probe mindestens die folgenden streng anaeroben Bakterien umfasst: *Bacteroides fragilis, Bacteroides nordii, Bacteroides thetaiotaomicron, Clostidium butyricum, Clostridium massilioamazoniensis, Clostridium beijerinckii, Clostridium irregular, Finegoldia magna, Propionibacterium acnes, Propionibacterium avidum, Haemophilus influenzae.*

10. Verfahren nach einem der Ansprüche 1 bis 9, **dadurch gekennzeichnet, dass** die eingefrorene Probe im Schritt c) während mindestens 12 h bei einer Temperatur von mindestens 0 °C und bei einem Hochvakuumdruck von nicht mehr als 1 mbar in einen Gefriertrockner gegeben wird.

11. Lyophilisat einer Probe einer Gruppe von aeroben und anaeroben Bakterien der Darmflora, die keine Milchsäurebakterien sind, das mit einem Verfahren nach einem der Ansprüche 1 bis 10 erhalten wird, **dadurch gekennzeichnet, dass** es umfasst:
- mindestens ein Schutzmittel, das mindestens aus der Kombination von Schutzmitteln besteht, die aus *(a1) einer Kombination von antioxidativen Verbindungen, die aus Ascorbinsäure, Harnsäure und Glutathion gewählt sind, a2) mindestens 2 verschiedenen Zuckern, die aus Saccharose und Trehalose bestehen, und a3) Milchproteinen bestehen,* und
- eine Menge lebender Bakterien, bevorzugt in einer Anzahl von:
- mindestens 10⁸ CFU/ml Bakterien jeder der Kategorien der aeroben Bakterien beziehungsweise anaeroben Bakterien, die nach 48 h Konservierung bei 4 °C kultiviert wurden, und
- mindestens 10⁸ CFU/ml Bakterien jeder der Kategorien der aeroben Bakterien beziehungsweise anaeroben Bakterien, die nach 6 Wochen Konservierung bei 4 °C kultiviert wurden.

12. Lyophilisat nach Anspruch 11, **dadurch gekennzeichnet, dass** die Probe eine klinische Probe von menschlichem Stuhl ist, wobei die Schutzmittel aus a1) einer Kombination von 3 antioxidativen Verbindungen, die aus Ascorbinsäure, Harnsäure und Glutathion bestehen, und a2) mindestens 2 verschiedenen Zuckern, die aus Saccharose und Trehalose bestehen, und a3) Milchproteinen bestehen.

## Claims

1. A method for preserving a sample of a set of aerobic and anaerobic bacteria other than lactic acid bacteria, in particular intestinal microbiota bacteria, said method comprising the following successive steps, wherein:
a) said sample of a set of said bacteria is prepared by means of dilution in an aqueous preservation medium comprising a combination of protective agents consisting of (a1) a combination of antioxidant compounds chosen from ascorbic acid, uric acid and glutathione and (a2) at least two different sugar consisting of sucrose and trehalose, and (a3) milk proteins, and
b) freezing said sample recovered in step a) in liquid nitrogen at approximately -196°C for no more than two minutes, preferably for 30 to 50 seconds, and
c) carrying out lyophilisation of said sample frozen in this way, in order to obtain a lyophilisate.

2. The method according to claim 1, **characterised in that** in step a), said sample is prepared which is a clinical stool sample by dilution of said stools in an aqueous preservation medium, and the dispersion obtained is filtered in order to remove at least the debris of size greater than 2 mm.

3. The method according to one of claims 1 to 2, **characterised in that** the milk proteins are in the form of whole milk, preferably at a concentration by weight in said preservation medium of at least 5%, preferably 10%.

4. The method according to one of claims 1 to 3, **characterised in that** said protective agents comprise a combination of antioxidant compounds consisting of ascorbic acid at a concentration of 1 g/L, uric acid at a concentration of 0.4 g/L and glutathione at a concentration of 0.1 g/L.

5. The method according to one of claims 1 to 4 **characterised in that** said protective agents comprise at least one said sugar at a concentration by weight in said preservation medium of at least 5%, preferably 10%.

6. The method according to one of claims 1 to 5, **characterised in that** said preservation medium is a buffer medium at pH of 7 to 7.5 comprising, in the water, the salts KCI, CaCl₂, MgCl₂, KH₂PO₄, Na₂HPO₄, NaCl and KOH, as well as at least one said protective agent.

7. The method according to one of claims 1 to 6, **characterised in that** said preservation medium comprises:
(a1) a combination of antioxidant compounds consisting of ascorbic acid at a concentration of 1 g/L, uric acid at a concentration of 0.4 g/L and glutathione at a concentration of 0.1 g/L, and
(a2) sucrose at a concentration by weight in said preservation medium of 5%, and trehalose at a concentration by weight in said preservation medium of 10%, and
(a3) milk proteins at a concentration by weight in said preservation medium of 10%.

8. The method according to one of claims 1 to 7, **characterised in that** said sample comprises at least the following aerobic bacteria and anaerobic bacteria: *Staphylococcus aureus, Enterobacter aerogenes, Escherichia coli, Klebsiella oxytoca, Streptococcus agalactiae, Serratia marcescens, Proteus mirabilis, Staphylococcus epidermidis, Bacillus circulans, Streptococcus pneumoniae, Staphylococcus hominis* and *Acinetobacter baumanii.*

9. The method according to one of claims 1 to 8, **characterised in that** said sample comprises at least the following strictly anaerobic bacteria: *Bacteroides fragilis, Bacteroides nordii, Bacteroides thetaiotaomicron, Clostidium butyricum, Clostridium massilioamazoniensis, Clostridium beijerinckii, Clostridium irregular Finegoldia magna, Propionibacterium acnes, Propionibacterium avidum, Haemophilus influenzae.*

10. The method according to one of claims 1 to 9, **characterised in that** in step c) the frozen sample is deposited in a freeze dryer for at least 12 hours at a temperature of at least 0°C and at a high vacuum pressure of not more than 1 mbar.

11. A sample lyophilisate of a set of aerobic and anaerobic intestinal bacteria other than lactic acid bacteria, obtained by a method according to one of claims 1 to 10, **characterised in that** it comprises:
- at least one protective agent consisting of at least the combination of protective agents consisting of (a1) a combination of antioxidant compounds chosen from ascorbic acid, uric acid and glutathione, (a2) at least two different sugar consisting of sucrose and trehalose, and (a3) milk proteins, and
- a quantity of live bacteria, preferably numbering:
- at least 10⁸ CFU/mL bacteria of each of the categories of aerobic bacteria and respectively anaerobic bacteria cultured after 48 hours preservation at 4°C, and
- at least 10⁸ CFU/mL bacteria of each of the categories of aerobic bacteria and respectively anaerobic bacteria cultured after 6 weeks preservation at 4°C.

12. A lyophilisate according to claim 11 **characterised in that** said sample is a clinical human stool sample, said protective agents consisting of a1) a combination of three antioxidant compounds consisting of ascorbic acid, uric acid and glutathione, and a2) at least two different sugars consisting of sucrose and trehalose, and a3) milk proteins.
